# EUROPEAN PATENT APPLICATION

(11) **EP 3 581 961 A1**
(43) Date of publication of application: **18.12.2019**
(21) Application number: 18177593.3
(22) Date of filing: 13.06.2018
(51) Int. Cl.: G01S 7/52, G01S 15/89, G10K 11/34

(54) **METHOD AND APPARATUS FOR ULTRASOUND IMAGING WITH IMPROVED BEAMFORMING**

(71) Applicant: Technische Universität München, 80333 München (DE)
(72) Inventor: HENNERSPERGER, Christoph, 84453 Mühldorf (DE); GÖBL, Rüdiger, 80689 München (DE); ZAHND, Guillaume, 80689 München (DE); SIMSON, Walter, 80807 München (DE)
(74) Representative: Lucke, Andreas

(57) **Abstract**

Disclosed herein is a method of ultrasound imaging of an object using an ultrasound transducer which comprises an array of transducer elements capable of converting sound signals into electrical signals and vice versa, comprising the following steps:
A) transmitting an ultrasound beam from said ultrasound transducer into the object, by activating a first subset of said transducer elements,
B) detecting reflected signals in a time resolved manner by means of a second subset of said transducer elements, wherein timing information of a detected signal is associated with information regarding the depth where the detected signal was reflected within the object subjected to imaging, and wherein the reflected signals associated with said second subset of transducer elements resemble a set of two-dimensional ultrasound data, of which one dimension resembles the various transducer elements of said second subset and the other dimension resembles depth information,
C) converting said two-dimensional ultrasound data into a scan object using a receive beamforming procedure which accounts for differences in distance of individual transducer elements from a given site of sound reflection within the object,
repeating steps A) to C) for different choices regarding at least one of said first and second subsets and the timing of the activation of transducer elements within said first subset, thereby obtaining a plurality of scan objects, and
a step of constructing a visual image from said plurality of scan objects,
wherein said receive beamforming procedure employs a machine learning based receive beamforming model for mapping said two-dimensional ultrasound data to said scan object.

## Description

### FIELD OF THE INVENTION

The present invention is in the field of ultrasound imaging. More particularly, the present invention relates to a method of ultrasound imaging employing novel beamforming procedures, as well as to an apparatus employing such method.

### BACKGROUND OF THE INVENTION

Ultrasound imaging is a well-established diagnostic modality that continuously undergoes tremendous development. Most of the basic research on ultrasound imaging in the early 20^{th} century was conducted with single transducer elements capable of converting sound signals into electrical signals and vice versa, where the transducer elements are typically formed by piezoelectric elements. Today's ultrasound transducers however usually consist of an array of transducer elements, typically within the range of 64 to 256 transducer elements. The array may be a 1D array for conventional imaging or a 2D array for so-called volumetric imaging.

Beamforming is employed in signal processing for both transmission and reception of acoustic waves to improve the directivity as well as the sensitivity of resulting data. In general terms, "beamforming" can be referred to as a technique to control electronic parameterization and signal transformation for the generation of ultrasound signals, also referred to as "transmit beamforming" herein, as well as to the processing of received and detected ultrasound signals to allow for a desired quality of the target signal ("receive beamforming"). Herein, the "quality" may refer to characteristics such as spatial resolution of distinguishable objects, signal-to-noise ratio, contrast or similar measures. An important aim of beamforming procedures is that by controlling the joint emission and reception of the array of transducer elements, the properties of the transmitted acoustic or electromagnetic waves can be optimized for specific locations in space such that the quality is increased.

A commonly used beamforming strategy is the so-called delay-and-sum beamforming technique, as e.g. described in Thomenius, ICE.: Evolution of ultrasound beamformers. In: IEEE Ultrasonics Symposium. Volume 2. IEEE (1996) 1615-1622*.* In delay-and-sum beamforming, in the transmit mode, a plurality of adjacent transducer elements are activated for generating a beam, where the activation of the transducer elements located on or close to the beam axis is delayed with regard to the activation of transducer elements further away from the beam axis. As a result of the transmit delays, the effective wavefront emitted into the tissue is optimized for focusing effects at a desired point in space, due to the compensation of varying times of arrival of the emitted pulses from each transducer element at the focal point. After receiving reflected signals from the tissue, a similar procedure can be applied in receive beamforming, where individual signals received by each transducer element are once again delayed and summed up for all receiving elements.

Delay-and-sum beamforming is currently the most common implementation for ultrasound beamforming. The main advantage of delay-and-sum beamforming is its relatively low computational complexity while providing reasonable image quality. In fact, due to the low computational complexity, both transmit and receive beamforming can be carried out in real time, i.e. while taking for example 50 ultrasound images per second.

In the art, there have been tremendous efforts to improve the image quality over images formed using delay-and-sum beamforming. For this purpose, advanced, data-dependent beamforming strategies have been proposed. An important example of advanced, data - dependent beamforming strategies is the so-called delay-multiply-and-sum method, as described e.g. in Matrone, G., Savoia, A.S., Galiano, G., Magenes, G.: The delay multiply and sum beamforming algorithm in ultrasound B-mode medical imaging. IEEE Transactions on Medical Imaging 34(4) (2015) 940-949*.* A further high-performance beamforming strategy is the Maximum Likelihood beamforming or Minimum Variance (MV) beamforming, as disclosed e.g. in Li, J., Stoica, P.: Robust adaptive beamforming. Volume 88. John Wiley & Sons (2005*).* Compared to the traditional data-independent delay-and-sum beamforming technique, these approaches yield a narrower main lobe and reduced side lobes of the received beams, improving both image resolution and contrast. However, such alternative approaches are hindered by a substantially higher computational cost, dramatically decreasing their real-time applicability.

In areas other than ultrasound imaging, learning approaches applied to multi-channel problems conceptually related to beamforming have been researched. For example, learning methods have been considered for significantly improving resulting signal quality in speech recognition. The conceptual relationship of these works as understood by the inventors relies on the fact that these works process complex, multi-channel audio data to a core signal, and the inventors found that insights gained from research applied to speech recognition are therefore also applicable to ultrasound imaging. Moreover, advantages of an end-to-end approach using convolutional neural networks (CNNs) on raw time-domain wave data, over frequency-domain, have first been demonstrated in the field of acoustic modeling, see e.g. Hoshen, Y., Weiss, R.J., Wilson, K.W.: Speech acoustic modeling from raw multichannel waveforms. In: IEEE International Conference on Acoustics, Speech and Signal Processing. (2015) 4624-4628*.* This approach was further explored in the context of an end-to-end speech recognition framework, as described in Sainath, T.N., Weiss, R.J., Wilson, K.W., Li, B., Narayanan, A., Variani, E., Bacchiani, M., Shafran, I., Senior, A., Chin, K., Misra, A., Kim, C.: Multichannel signal processing with deep neural networks for automatic speech recognition. IEEE/ ACM Transactions on Audio, Speech, and Language Processing 25(5) (2017) 965-979*.* CNN-supported beamforming techniques have been proposed by Xiao, X., Watanabe, S., Erdogan, H., Lu, L., Hershey, J., Seltzer, M.L., Chen, G., Zhang, Y., Mandel, M., Yu, D.: Deep beamforming networks for multi-channel speech recognition. In: IEEE ICASSP. (2016) 5745-5749*,* to estimate the optimal beamforming parameter settings.

In Yoon, Y.H., Ye, J.C.: Deep learning for accelerated ultrasound imaging. arXiv preprint arXiv:1710.10006 (2017*),* CNNs have been applied to sparse raw channel data within a compressed-sensing framework, in order to fill in missing signals. However, in these studies, learning was applied for pre-beamforming tasks, such as parameter-optimization and signal reconstruction, but the beamforming *per se* was performed using a conventional implementation.

In the art, there remains a need for ultrasound imaging with high image quality without sacrificing real-time imaging capabilities.

### SUMMARY OF THE INVENTION

The problem underlying the invention is to provide a method and an apparatus for ultrasound imaging of an object which allows for improved ultrasound image quality at high processing speeds.

This problem is solved by a method according to claim 1 and by an apparatus according to claim 9. Preferable embodiments are defined in the dependent claims.

According to one aspect of the invention, a method of ultrasound imaging of an object is provided, using an ultrasound transducer which comprises an array of transducer elements capable of converting sound signals into electrical signals and vice versa. The method comprises the following steps:
A) transmitting an ultrasound beam from said ultrasound transducer into the object, by activating a first subset of said transducer elements,
B) detecting reflected signals in a time resolved manner by means of a second subset of said transducer elements, wherein timing information of a detected signal is associated with information regarding the depth where the detected signal was reflected within the object subjected to imaging, and wherein the reflected signals associated with said second subset of transducer elements resemble a set of two-dimensional ultrasound data, of which one dimension resembles the various transducer elements of said second subset and the other dimension resembles depth information,
C) converting said two-dimensional ultrasound data into a scan object using a receive beamforming procedure which accounts for differences in distance of individual transducer elements from a given site of sound reflection within the object,
repeating steps A) to C) for different choices regarding at least one of said first and second subsets and the timing of the activation of transducer elements within said subset, thereby obtaining a plurality of scan objects, and
a step of constructing a visual image from said plurality of scan objects,
wherein said receive beamforming procedure employs a machine learning based receive beamforming model for mapping said two-dimensional ultrasound data to said scan object.

Herein, the term "two-dimensional ultrasound data" shall be understood to be *at least* two-dimensional, i.e. it could be of precisely two dimensions or of higher dimensions. Moreover, the "scan object" could for example be a scan line, which represents sound reflection at various depths along a line extending from said transducer, and in particular from a given transducer element of the transducer, into the object subjected to imaging. However, other types of scan objects are likewise possible.

Moreover, the term "first/second subset of transducer elements" may refer to any selection of transducer elements within the array, including all transducer elements within the array, and is in particular not limited e.g. to subsets of directly adjacent transducer elements or the like.

The inventors could confirm that by employing a machine learning based receive beamforming model for mapping said two-dimensional ultrasound data to said scan object, ultrasound images of very high quality can be obtained in real time, once the beamforming model has been properly trained using machine learning. Indeed, it is seen that the quality of the ultrasound images is mainly limited by the quality of the training data, and hence the beamforming that was applied in the training data. Accordingly, the receive beamforming model can be trained using image data obtained with sophisticated, computationally expensive beamforming methods that would be prohibitive for real-time ultrasound imaging, and similar image quality can be obtained with a fully trained model. In other words, all of the explicit formulations of the sophisticated beamforming processes, such as "delay-multiply-and-sum" or "Minimum Variance" beamforming, which are responsible for high computational time, are suppressed in the receive beamforming model of the invention and replaced by an implicit inferring step. The inferring step is completely independent of the mimicked sophisticated beamforming method in terms of its computational speed, and can require as little as 0.03 seconds per image in a current embodiment of the invention.

In a preferred embodiment, said machine learning based receive beamforming model employs one of a deep convolutional neural network or a recurrent neural network. The inventors have found out that both, recurrent neural networks and deep convolutional neural networks are particularly suitable for creating a receive beamforming model for the purposes of the invention. Indeed, both, recurrent neural networks and deep convolutional networks have the ability to learn custom spatial and temporal filters of an arbitrary dimension over the ultrasound data in order to create a mapping to beamformed scan data. Furthermore, representations of ultrasound data which display inferred contextual and diagnostic information may also be generated. One example of such "diagnostic information" is a spatial distribution of speed of sound, as is explained in more detail below. The use of recurrent and convolutional neural network based filters allows for the steps to be concurrently performed in an optimal manner in a learning context.

In a preferred embodiment, said receive beamforming model is an end-to-end beamforming model receiving said two-dimensional ultrasound data as an input and directly converting it into said scan object.

In an alternative embodiment, said receive beamforming model receives said two-dimensional ultrasound data and maps it onto a set of delay values and weight values for use in a delay-and-sum receive beamforming algorithm. Indeed, the performance of ordinary delay-and-sum receive beamforming can be considerably improved if the delay values and weight values are properly chosen. In particular, in conventional delay-and-sum beamforming, the delays are calculated based on the assumption of a constant speed of sound in human tissue, which is generally assumed to be 1540 m/s. However, upon a closer look, it is found that depending on the exact nature of the imaged tissue, this value can indeed vary as much as +/- 7%, and as a result of this, the delay values neglecting these variations are not precise, which the inventors believe to be the main source of errors in conventional delay-and-sum beamforming. However, using a machine learning based beamforming model, more appropriate delay values and also improved weight values can be generated from said two-dimensional ultrasound data and then fed into a per se known delay-and-sum receive beamforming algorithm. Note that as before, said "two-dimensional ultrasound data" means that this data is "at least two-dimensional". Accordingly, a step of "receiving said two-dimensional ultrasound data and mapping it onto a set of delay values and weight values" does not e.g. rule out a scenario in which spatial similarity of neighbouring scan objects is used by employing 3D convolution filters, for example by filtering over a 3D collection of 3D scan objects to gain greater confidence about the generated image.

Note that in both variants, the end-to-end beamforming model or the beamforming model mapping two-dimensional ultrasound data into a set of delay values and weight values for use in a delay-and-sum receive beamforming algorithm have an implicit or encoded "understanding" of the speed of sound within the object, since they allow for compensating the effects of a deviation of the true, local speed of sound from an assumed, constant global speed of sound. Moreover, in a preferred embodiment, said receive beamforming model is further configured to determine a spatial distribution of speed of sound within the object. In other words, in this variant, the beamforming does not only make implicit use of information regarding the spatially dependent speed of sound, but determines it explicitly. In a preferred embodiment, the method further comprises indicating speed of sound related information in the visual image. This speed of sound related information allows for augmenting the ultrasound image with further useful information. Namely, different tissue types, such as fat, blood, muscle, vascular structures, myocardial tissue, et cetera have specific, slightly different and well characterized speed of sound. Therefore, generating an image in which a precise speed of sound information is known for each pixel enables immediate and automatic identification of different types of tissues in the entire image. In preferred embodiments, the machine learning based beamforming model carries out tissue clustering and boundary classification, which again can be well implemented in machine learning, in particular, employing deep convolutional neural networks or recurrent neural networks.

To the knowledge of the inventors, automatic speed of sound retrieval for specific regions in the tissue does not exist yet in ultrasound imaging. However, this task can be achieved with preferred embodiments of the machine learning based beamforming models of the invention. For this purpose, tissues can be insonified from different angles and the variance between receive signals originating from the same point in space can be minimized. This means that additional information may be used to produce an image. The same location may be imaged from several angles of incidence. The gray-level value of every voxel in space may be determined in an optimal fashion, not by simply averaging the collection of acquired planes, but by first determining the optimal delay and weight coefficients for each channel in such way that the variance is minimized.

In a preferred embodiment, one or both of said first and second subsets of transducer elements corresponds to a number of transducer elements within a predefined aperture region centered at a given transducer element.

In preferred embodiments, said first and second subsets of transducer elements may overlap with each other, wherein preferably at least 50%, more preferably at least 75% of the transducer elements in one of said first and second subsets is also part of the other one of said first and second subsets, wherein said first and second subsets are most preferably identical.

In some embodiments, the first subset of transducer elements may be larger than the second subset of transducer elements. In some embodiments, the same first subset may be combined with different second subsets. For example, the first subset may even correspond to the entire array of transducer elements, i.e. a "full aperture", while different second subsets are used as receive channels for receive beamforming.

As was mentioned above, the "scan object" may be a scan line, representing sound reflection at various depths along a line extending from said transducer, in particular from a given transducer element of said transducer, into the object subjected to imaging.

In a preferred embodiment, said step of constructing a visual image from said plurality of scan objects comprises one or more of a demodulation, in particular a demodulation via envelope detection, a logarithmic compression and a scan conversion/re-interpolation. Herein, scan conversion can for example involve a process of taking linear data and interpolating the same into a different coordinate system. This could for example involve interpolating scan lines that can be acquired on a fan to a rectangular grid, or the like.

In a preferred embodiment, said machine learning based receive beamforming model has been trained, or is obtainable by training using training data obtained with different conventional receive beamforming methods, and in particular with one or more of delay-and-sum beamforming, delay-multiply-and-sum and minimum variance beamforming.

In particular, said machine learning based receive beamforming model may have been trained, or obtainable by training, in a procedure, in which
- two or more receive beamforming procedures are carried out on the same two-dimensional ultrasound data but using different conventional receive beamforming methods, leading to a corresponding number of different scan objects, and wherein a resultant scan object is selected or derived from said plurality of different scan objects, and
- the training is carried out based on said resultant scan object.

This embodiment accounts for the fact that the performance of a machine learning based receive beamforming model is dependent on and hence limited by the quality of the underlying training data. This means that when training the machine learning based receive beamforming model using data generated with a single conventional receive beamforming method, it will eventually mimic its behavior both, with regard to its strengths, but also with regard to its deficiencies. According to this embodiment, it is suggested to carry out a plurality of beamforming procedures on the same two-dimensional ultrasound data but using different conventional receive beamforming methods, which then leads to a corresponding number of different scan objects. Based for example on an automatically retrieved quality metrics, such as ultrasound confidence maps, image statistics or the like, a resultant scan object can then be selected or derived from said plurality of different scan objects, which exhibits the best quality. The training is then carried out based on this resultant scan object, which means that the thus obtained machine learning based receive beamforming model combines the advantages and strengths of various different conventional beamforming methods in one model.

In a particularly preferred embodiment, in step A), the activation of said first subset of transducer elements, and in particular a relative weight and/or a relative delay of their activation, is controlled using a transmit beamforming procedure employing a machine learning based transmit beamforming model that has been trained, or is obtainable by training in combination with said machine learning based receive beamforming model, and that receives, as at least part of its input, said two-dimensional ultrasound data or said scan objects.

According to this embodiment, the machine learning is hence not only employed on the receive side, but also for transmit beamforming. This allows for a yet further significant increase in imaging quality. In particular, using machine learning, the transmit beamforming model can implicitly learn from the two-dimensional ultrasound data or scan objects information related to the location dependent speed of sound and use this implicit knowledge in an optimum transmit beamforming leading to optimal image quality, in combination with the receive beamforming with which the transmit beamforming has been trained.

In an alternative embodiment, the activation of said first subset of transducer elements, and in particular a relative weight and/or a relative delay of their activation is controlled using information regarding a spatial distribution of speed of sound within the object determined by means of said receive beamforming model. This variant exploits the fact that along with or as part of the machine learning based receive beamforming, information regarding the spatial distribution of speed of sound in the object to be imaged can be obtained. Once this information is available, it can be exploited in transmit beamforming, where the delays of the activation of individual transducer elements within this first set of transducer elements can be precisely adjusted such that the ultrasound pulses generated by the individual transducer elements arrive simultaneously at the envisaged focal point.

A further aspect of the invention relates to an apparatus for ultrasound imaging of an object. The apparatus comprises ultrasound transducer which comprises an array of transducer elements capable of converting sound signals into electrical signals and vice versa. The apparatus further comprises a control unit, wherein said control unit is configured for controlling the apparatus to carry out the following steps:
A) transmitting an ultrasound beam from said ultrasound transducer into the object, by activating a first subset of said transducer elements,
B) detecting reflected signals in a time resolved manner by means of a second subset of said transducer elements, wherein timing information of a detected signal is associated with information regarding the depth where the detected signal was reflected within the object subjected to imaging, and wherein the reflected signals associated with said second subset of transducer elements resemble a set of two-dimensional ultrasound data, of which one dimension resembles the various transducer elements of said second subset and the other dimension resembles depth information,
C) converting said two-dimensional ultrasound data into a scan object using a receive beamforming procedure which accounts for differences in distance of individual transducer elements from a given site of sound reflection within the object,
repeating steps A) to C) for different choices regarding at least one of said first and second subsets and the timing of the activation of transducer elements within said first subset, thereby obtaining a plurality of scan objects, and
a step of constructing a visual image from said plurality of scan objects,
wherein said receive beamforming procedure employs a machine learning based receive beamforming model for mapping said two-dimensional ultrasound data to said scan object.

In a preferred embodiment, said machine learning based receive beamforming model employs one of a deep convolutional neural network or a recurrent neural network.

In a preferred embodiment, said receive beamforming model is an end-to-end beamforming model receiving said two-dimensional ultrasound data as an input and directly converting it into said scan object.

In an alternative embodiment, said receive beamforming model is configured to receive said two-dimensional ultrasound data and to map it onto a set of delay values and weight values for use in a delay-and-sum receive beamforming algorithm.

The receive beamforming model may further be configured to determine a spatial distribution of speed of sound within the object, wherein the control unit may further be configured for indicating speed of sound related information in the visual image.

In a preferred embodiment, one or both of said first and second subsets of transducer elements corresponds to a number of transducer elements within a predefined aperture region centered at a given transducer element. Preferably, said first and second subsets of transducer elements overlap with each other, wherein preferably at least 50%, more preferably at least 75% of the transducer elements in one of said first and second subsets is also part of the other one of said first and second subsets. In particular, said first and second subsets may be identical.

In some embodiments, the first subset of transducer elements is larger than the second subset of transducer elements. Herein, a same first subset is preferably combined with different second subsets. For example, the first subset may correspond to the entire array of transducer elements, while different second subsets may be used as receive channels for receive beamforming.

In a preferred embodiment, said scan object is a scan line, representing sound reflection at various depths along a line extending from said transducer, in particular from a given transducer element of said transducer, into the object subjected to imaging.

In a preferred embodiment, said step of constructing a visual image from said plurality of scan objects comprises one or more of a demodulation, in particular a demodulation via envelope detection, a logarithmic compression and a scan conversion/re-interpolation.

In various embodiments, said machine learning based receive beamforming model has been trained, or is obtainable by training using training data obtained with different conventional receive beamforming methods, and in particular with one or more of delay-and-sum beamforming, delay-multiply-and-sum and minimum variance beamforming.

In a preferred embodiment, said machine learning based receive beamforming model has been trained in a procedure or is obtainable by training in a procedure, in which
- two or more receive beamforming procedures are carried out on the same two-dimensional ultrasound data but using different conventional receive beamforming methods, leading to a corresponding number of different scan objects, and wherein a resultant scan object is selected or derived from said plurality of different scan objects, and
- the training is carried out based on said resultant scan object.

In a preferred embodiment, said control unit is configured to control in step A) the activation of said first subset of transducer elements, and in particular a relative weight and/or a relative delay of their activation,
- using a transmit beamforming procedure employing a machine learning based transmit beamforming model that has been trained in combination with said machine learning based receive beamforming model, and that receives, as at least part of its input, said two-dimensional ultrasound data or said scan objects, or
- using information regarding a spatial distribution of speed of sound within the object determined by means of said receive beamforming model.

### SHORT DESCRIPTION OF THE FIGURES

- Fig. 1: schematically shows an ultrasound transducer and a beam formed thereby.
- Fig. 2: schematically shows the beamforming workflow according to an embodiment of the invention.
- Fig. 3: is a schematic representation of a data flow in an end-to-end beamforming procedure.
- Fig. 4: schematically shows the architecture of a deep convolutional neural network for use in receive beamforming.
- Fig. 5: schematically shows a residual block with batch normalization between convolutional layers of the neural network of Fig. 4.
- Fig. 6: is a conceptual dataflow diagram showing the generation of optimized scan lines by applying different conventional beamforming methods to the same two-dimensional ultrasound data.
- Fig. 7a: is a schematic representation of a speed of sound deepformer.
- Fig. 7b: is a schematic representation of an alternative speed of sound deepformer.
- Fig. 8: shows a comparison of different beamforming strategies based on the Structural Similarity Index Measure (SSIM).
- Fig. 9: shows representative examples of the similarity between deepformed images and corresponding delay-and-sum and minimum variance ground truth images.
- Fig. 10: shows examples of the point spread function obtained using a wire phantom.
- Fig. 11: shows images demonstrating how modifications of the ultrasound scanner parameter settings affect the deepformed images.

### DESCRIPTION OF THE PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

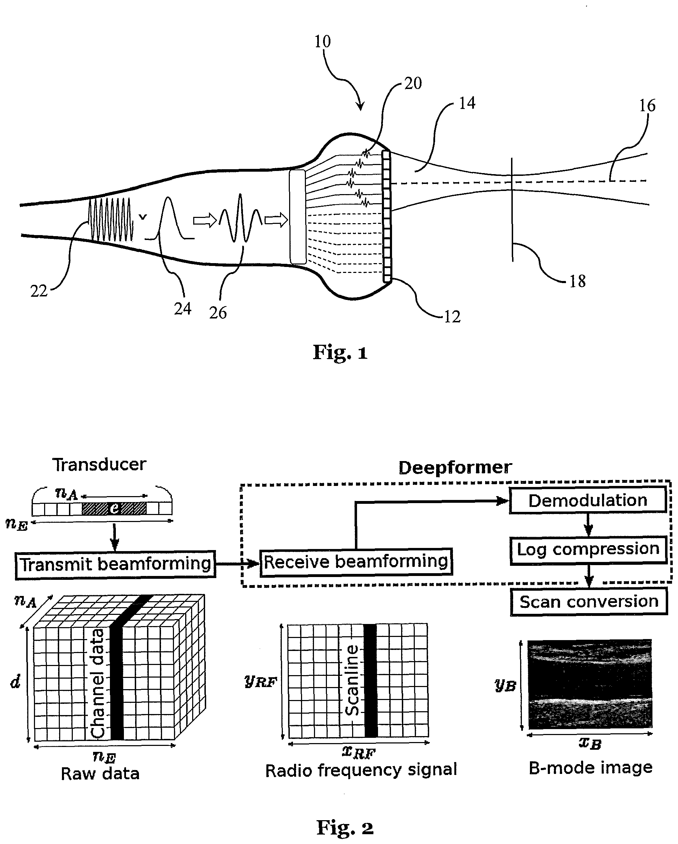

Fig. 1 schematically shows the working principle of an ultrasound transducer 10 for use in a method and system according to the invention. The exemplary ultrasound transducer 10 shown in Fig. 1 comprises a linear array of *n_{E}* transducer elements 12 which are capable of converting sound signals into electrical signals and vice versa. In the embodiment shown, the transducer elements 12 are piezoelectric elements.

Further schematically shown in Fig. 1 is an ultrasound beam 14 having a beam axis 16, wherein said beam 14 is focused in a focal region indicated with reference sign 18. In order to generate such a focused ultrasound beam 14, a subset of transducer elements 12, referred to as the "first subset" herein, is activated by means of corresponding electrical pulses 20. The first subset of transducer elements 12 is also referred to as "aperture" or "aperture region" of the transducer array herein.

For generating the individual activation pulses 20 for individual transducer elements 12, a carrier signal 22 is combined with a shape signal 24 such as to generate a main pulse 26. The individual activation pulses 20 are derived from the main pulse 26. As is seen in the schematic representation of Fig. 1, individual pulses 20 close to the beam axis 16 are delayed with respect to pulses 20 further away from the beam axis 16 such that the partial soundwaves (not shown) emitted by the individual transducer elements 12 arrive approximately simultaneously in the focal region 18 on the beam axis 16. The activation of the individual transducer elements 12 within the aperture is referred to as "transmit beamforming". In conventional beamforming, a universal speed of sound is assumed for the tissue subjected to imaging, and based on this assumption, suitable delays can be calculated in a straightforward manner based on Pythagoras' rule. However, a closer inspection reveals that the speed of sound actually varies throughout the tissue, and the inventors have noticed that by not accounting for this inhomogeneous speed of sound distribution, simple, data independent beamforming methods such as the delay-and-sum beamforming method only allow for limited ultrasound imaging quality.

The thus generated ultrasound pulse will be reflected at the tissue and reflected signals are detected in a time resolved manner by means of a second subset of said transducer elements 12, which may, but need not be the same as the first subset. Herein, the timing information of the detected signal is associated with information regarding the depth in which the detected signal was reflected within the object subjected to imaging. Accordingly, the reflected signals associated with the second subset of transducer elements resemble essentially a set of two-dimensional ultrasound data, of which one dimension resembles the various transducer elements of said second subset and the other dimension resembles depth information. The two-dimensional ultrasound data are then converted into a scan object. In the embodiment shown, the scan object is a scan line, associated with the beam axis 16, representing sound reflection at various depths along said beam axis 16. The conversion of the two-dimensional ultrasound data into the scan object is referred to as receive beamforming herein.

The beamforming workflow according to an embodiment of the invention is shown in more detail with reference to Fig. *2**.* As mentioned with reference to Fig. 1, the transducer 10 comprises an array of *n_{E}* piezoelectric elements 12 that are able to transmit and receive signals. A transmission phase (TX) and a reception phase (RX) are repeated alternatively *n_{E}* times, once for each element e ∈ {1, ... , *n_{E}*} of the transducer 10, by shifting the aperture window over the transducer array, thereby selecting the aforementioned "first subset" of transducer elements 12.

During transmission, an ultrasound pulse of a given frequency is propagated in the tissues through electrical excitation of *n_{A}* aperture elements centered around the element e. The direction and focal depth of the propagated wave is controlled through transmit beamforming as explained above. After propagation and reflection in the tissue, the received signals impinging on the transducer 10 constitute the raw channel data, of size *n_{A}* x d, which is an example of the "two-dimensional ultrasound data" referred to above.

During receiving, a receive beamforming operation is carried out for converting the two-dimensional ultrasound data into a scan object. The receive beamforming procedure effectively accounts for differences in distance of individual transducer elements 12 from a given site of sound reflection within the object. This way, a 1D scan line, or radio frequency signal (RF, size 1 x *y_{RF}*) is obtained. Finally, to create a visually interpretable B-mode (i.e. "brightness mode") image, the RF signal undergoes demodulation, for example via envelope detection, a log compression, to improve the dynamic range, and a scan conversion, which may comprise a re-interpolation from x*_{RF}* × *y_{RF}* to x*_{B}* × y*_{B}.*

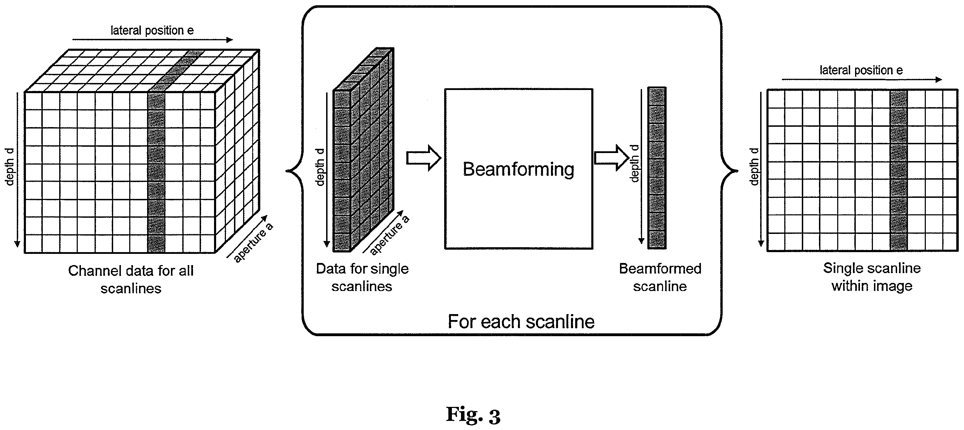

While the receive beamforming procedure accounts for differences in distance of individual transducer elements 12 from the reflection site, in the end-to-end beamforming version of the invention, this is not carried out by introducing explicit delays to the received signals or the like. Instead, the receive beamforming procedure employs a machine learning based receive beamforming model for mapping the two-dimensional ultrasound data to the scan line. Since this process combines beamforming with deep learning, the process is also referred to as "deepforming" herein, and the model is referred to as a "deepformer". The deepformer may be trained to map the receiving, demodulation, and log compression steps of the ultrasound image formation pipeline as shown in Fig. 2*.* Given a raw scan line X ∈ R^{d×nA} and a fully processed beamformed scan line Y ∈ R^{1×yRF}, the deepforming pipeline *BF* is achieved by learning the transformation *BF(X) -> Y.* This embodiment hence employs an end-to-end deepforming, in which the two-dimensional ultrasound data is received as input and it is directly converted into the scan line. A straightforward operation is then finally applied to concatenate all the deep formed arrays and reshape them in the dimension of the visually understandable final image. The specific data flow of the beamforming part is further illustrated in Fig. 3. The reason why this last operation is provided after the deepforming is twofold. The first reason is that the reshape operation is very simple, as essentially only a resize operation is conducted. The second reason is that it is more robust to apply the deepformer to each channel data and to obtain a single factor, rather than extracting several image columns from a unique initial channel data.

The machine learning based receive beamforming models may employ one of a convolutional neural network or a recurrent neural network. As was pointed out above, the inventors have noticed that both, recurrent neural networks and deep convolutional neural networks are particularly suitable for creating a receive beamforming model for the purposes of the invention. One of the reasons for this is that recurrent neural networks and deep convolutional networks have the ability to learn custom spatial and temporal filters of an arbitrary dimension over the ultrasound data in order to create a mapping to beamformed scan data. Furthermore, representations of ultrasound data which display inferred contextual and diagnostic information may also be generated. The use of recurrent and convolutional neural network based filters allows for the steps to be concurrently performed in an optimal manner in a learning context.

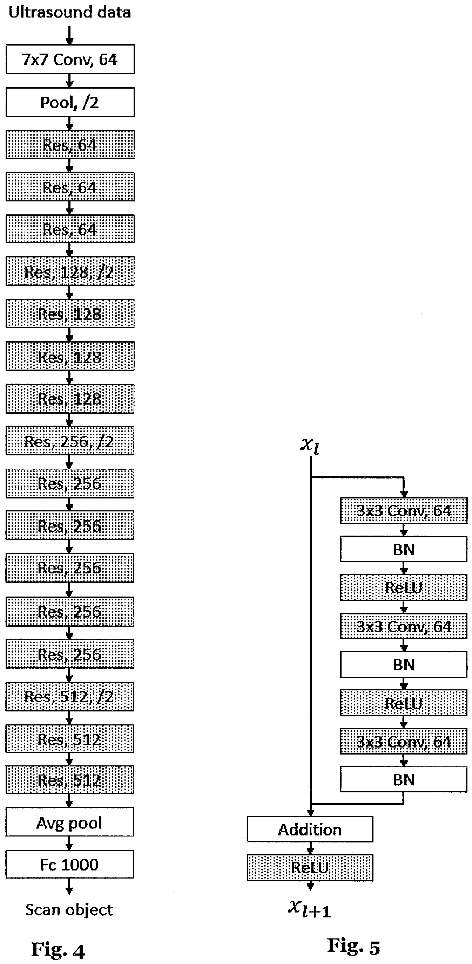

In one embodiment, the neural network employed for the end-to-end deepformer is a deep convolutional neural network with residual skip connections and 4 bottlenecks. The network used in a currently operative version uses 50 total layers. In Fig. 4, a 34 layer exemplary architecture diagram is shown. The currently employed end-to-end deepformer implementation employs batch normalization between convolutional layers of the residual blocks as illustrated in Fig. 5.

The blocks each describe computations performed on their inputs as indicated by the directed connections (the "arrows"). If an operation is labeled as "/2", the operation is applied to every second pixel, resulting in an output of half the size.

The "Conv" blocks apply filters of the specified size (e.g. 3x3 pixels), "Pool" and "Avg pool" accumulate their input in a small spatial window, thereby performing smoothing. Blocks called "Res" are so called residual blocks, their structure is shown in Fig. 5. The "BN" blocks perform a process called batch normalization, in which its inputs are scaled based on statistics observed during training time. This improves the numeric stability of the overall process. "ReLU" blocks introduce non-linearity to the network, by rectifying their inputs, in the example shown, they compute max(o, x). And finally, the block "FC" is a layer in the neural network that has d outputs. In this layer, all inputs are connected to each output with a learned weight, effectively computing d linear combinations of its input.

The input "ultrasound data" as depicted in Fig. 4 is raw ultrasound data of dimension [aperture-size ×depth], i.e. [*n_{A}* x d] (cf. Fig. 2). The output size of the fully connected layer is [1 × depth scanline].

As was indicated above, the performance of receive deepforming is limited by the quality of the underlying training data. When training the deepformer using data generated with a single conventional receive beamforming method, the deepformer will eventually mimic its behavior both, with regard to its strengths, but also with regard to its deficiencies. In a sophisticated implementation of deepforming as an end-to-end receive deepformer, for obtaining optimum training data, the output of multiple advanced conventional beamforming can be associated with a quality metric that can mathematically dictate the properties of an output image, for example color map, contrast etc. This way, an optimized scanline can be constructed based on any given two-dimensional ultrasound input data, using the strengths of various existing advanced beamforming methods, and this optimized scanline, together with the two-dimensional ultrasound data, can be used for training the deepformer.

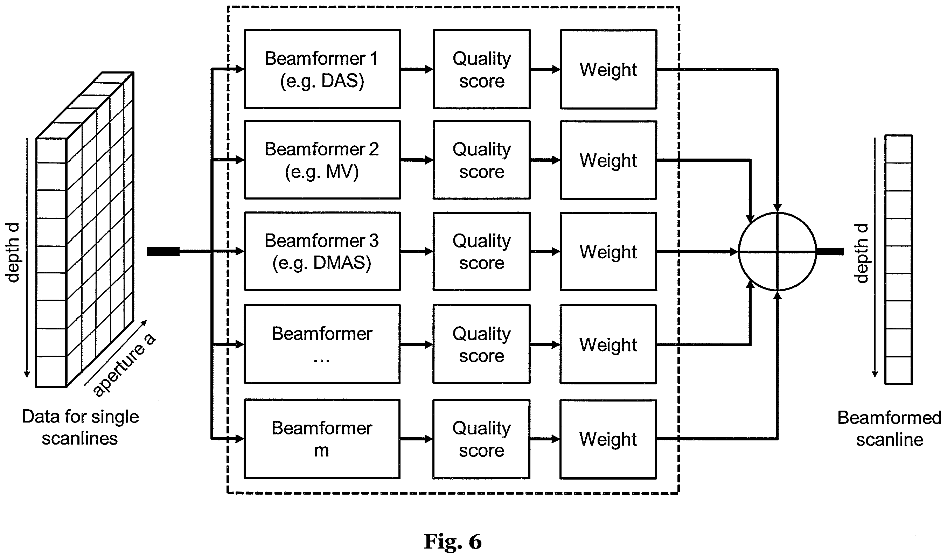

A conceptual dataflow diagram is shown in Fig. 6. As indicated therein, the same two-dimensional ultrasound input data are subjected to different forms of conventional beamforming, such as delay-and-sum, minimum variance, delay-multiply-and-sum, or the like. Each of the conventional beamformers generate a corresponding beamformed scanline. Each of the beamformed scan lines is subjected to some quality metric awarding a quality score thereto. The quality metrics may involve automatically retrieved quality metrics, such as ultrasound confidence maps, image statistics or the like. However, at least some contribution to the quality score could also be based on expert input based on finally reconstructed images. If the quality metric indicates a high quality standard, a high weight factor is associated with the beamformed scanline, and vice versa. Then, a weighted sum of the individual beam formed scanlines is computed, which resembles an example of the "resultant scan object" referred to above, and which is derived from the set of beamformed scanlines obtained with different conventional beamformers. In a simple embodiment, the beam formed scanline having the highest quality score can simply be selected, which would correspond to assigning a weight factor of 1 to the beamformed scanline with the highest quality score and a weight factor of o to the other scanlines. However, in general the resultant scanline will be the weighted sum of individual scanlines. The training is then carried out based on this resultant beamformed scanline, which means that the thus obtained machine learning based receive beamforming model combines the advantages and strengths of various different conventional beamforming methods in one model.

As was explained above, one underlying assumption for all currently practiced beamforming approaches is a constant and predefined speed of sound in human tissue, amounting to 1540 m/s in most medical systems. This assumption is not only used for transmit beamforming, but also in all prior art receive beamforming methods known to the inventors. By modelling the receive beamforming and wave propagation into tissue as a neural network, a specific speed of sound value can be employed as a characteristic value to automatically optimize receive delays in the deepformer. The idea is that a given parameterization of the speed of sound for a received set of signals will for example minimize the variance and thus maximize image quality.

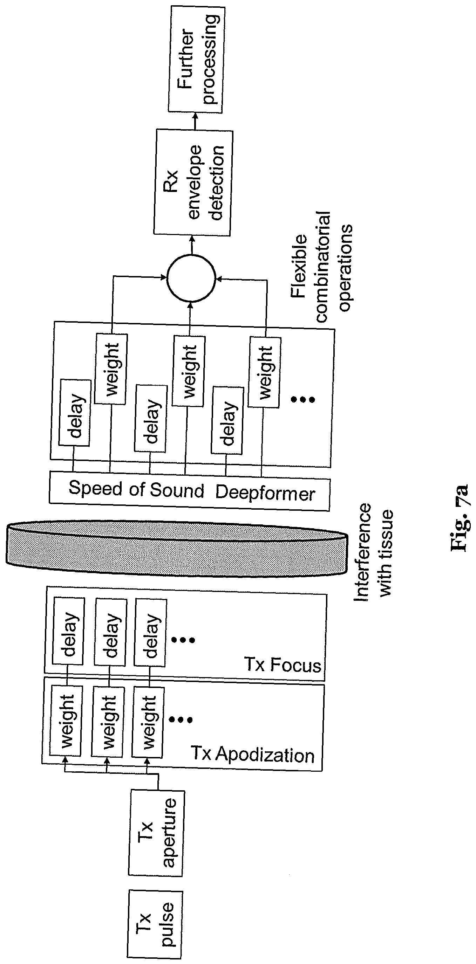

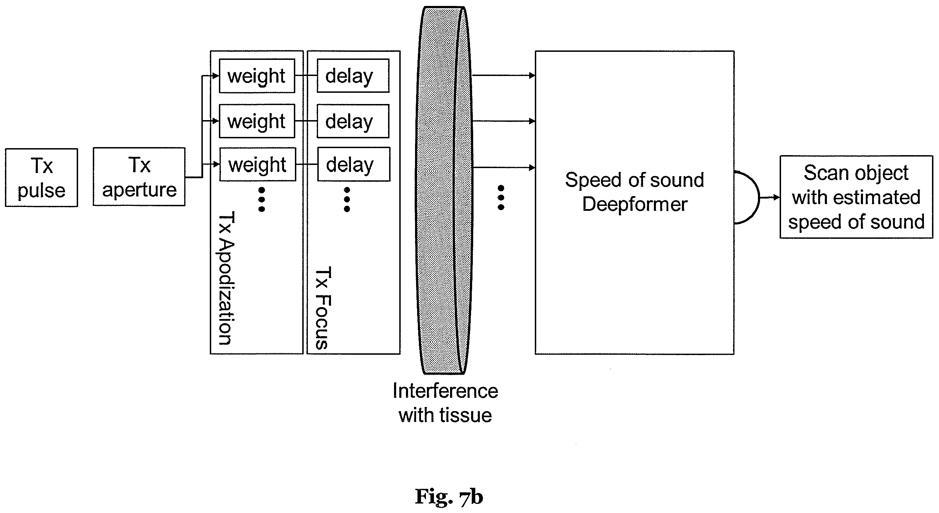

Fig. 7a is a schematic representation of a corresponding type of deepformer which is referred to as a "speed of sound deepformer" herein. It represents a machine learning based receive beamforming model which receives the two-dimensional ultrasound data and maps it onto a set of delay values and weight values for use in a delay-and-sum receive beamforming algorithm. This deepformer is referred to as a "speed of sound deepformer", because the optimum delays obtained by it implicitly reflect the precise distribution of speed of sound in the tissue, and in particular reflect any deviation from the typical assumption of a uniform speed of sound at 1540 m/s. However, in a further variant shown in Fig. 7b, the speed of sound deepformer may explicitly determine estimated speed of sound at each location within the tissue. In some embodiments, as e.g. shown in Fig. 7b, this local speed of sound information may be outputted. For example, in one embodiment, the speed of sound related information may be indicated in the visual image, or be used to augment the visual image. For example, since different types of tissues will typically have different speeds of sound, that speed of sound information can help to improve the contrast of the ultrasound images, and thereby may be used for augmenting the ultrasound images.

While in the embodiment shown in Fig. 2, the deepforming has been applied only for receive beamforming, in a preferred embodiment, transmit beamforming is likewise based on a machine learning based model. For this purpose, in step A), the activation of said first subset of transducer elements, and in particular a relative weight and/or a relative delay of their activation, is controlled using a transmit beamforming procedure employing a machine learning based transmit beamforming model that has been trained, or is obtainable by training in combination with said machine learning based receive beamforming model, and that receives, as at least part of its input, said two-dimensional ultrasound data or said scan objects. According to this embodiment, the machine learning is hence not only employed on the receive side, but also for transmit beamforming. This allows for a yet further significant increase in the imaging quality. In particular, using machine learning, the transmit beamforming model can implicitly learn from the two-dimensional ultrasound data or scan objects information related to the location dependent speed of sound, and use this implicit knowledge in an optimum transmit beamforming leading to optimal focusing and hence improved image quality, in combination with receive beamforming with which transmit beamforming has been trained.

In an alternative embodiment, the activation of the first subset of transducer elements, and in particular a relative weight and/or a relative delay of their activation is controlled using information regarding the spatial distribution of speed of sound within the object determined by means of receive beamforming, for example by means of a variant of the speed of sound beamformer described above in a variant that allows for providing explicit speed of sound information.

Next, results obtained with end-to-end beamforming of the type shown in Fig. 2 and 3 are presented. The network used for this end-to-end beamforming was built on the residual network (ResNet) architecture and consisted of 50 layers comprised of 4 blocks, of 3 residual units each with intermediate normalization layers. Due to the strong correlation between the raw input signals and the fully processed beamformed output, the identity mapping connections in ResNet allow for the propagation of important features to distant layers, while convolution filters can readily approximate the temporal and spatial integration of beamforming. For further details of the residual network architecture, reference is made to He, K., Zhang, X., Ren, S., Sun, J.: Identity mappings in deep residual networks. In: European Conference on Computer Vision. (2016) 630-645*.*

The network was implemented in Python and Tensorflow v1.5. Training was performed using the Adam optimizer (cf. Kingma, Diederik P., and Jimmy Ba. "Adam: A method for stochastic optimization." arXiv preprint arXiv:1412.6980 (2014)) with an initial learning rate of 0.001 and the mean squared error (MSE) loss function. The loss was computed between the output of the network and a fully processed scan line of the same dimension. Training was completed with early-stopping regularization. To enforce the consistency of the scan line data along the axial dimension, the input to the network was the raw data for a full scan-line, i.e. a 2D structure spanned by the depth and the aperture. To cope with the variance in the raw data caused by attenuation and time gain compensation, both input and output data were normalized depth-wise. This operation was conducted by calculating the means and standard deviations at equal depths of all the processed scan lines in the training set, followed by normalization to zero mean and unit standard deviation.

Two networks of the same architecture were trained on beamformed scan-line data, processed using conventional delay-and-sum beamforming (DS in the following) and conventional minimum variance beamforming (MV), respectively. Experimental inference was performed on a NVIDIA TITAN Xp GPU (Pascal architecture) with an average inference time of 0.9364 ± 0.0055 s per image.

Data acquisition ultrasound imaging was performed using a publicly available beamforming software pipeline (SUPRA) with a cQuest Cicada scanner (Cephasonics, CA, USA) equipped with a 128 elements linear transducer (CPLA12875,7 MHz). The imaging depth was set to 40 mm, pulse frequency of 7 MHz with a single focus at 20 mm, and the dynamic range was 50 dB. Ground truth B-mode images were systematically generated using the previously collected raw ultrasound data via SUPRA's DS and MV beamformers. The dimension of the resulting data was as follows: raw data, (d × *n_{E}* ×*n_{A}*) = (2077 × 256 × 64); RF signal, (*x_{RF}*×*Y_{RF}*) = (256 × 2000); B-mode image, (*x_{B}* × *y_{B}*) = (753 x 800), corresponding to 37.5 x 40.0 mm².

In vivo ultrasound images were acquired on 22 healthy participants (age: 32±13 years, 14 males). All scans were performed for both sides (left, right) and both orientations (longitudinal, transverse), for a duration of at least 10 s (yielding a minimum of 19 frames per scan), while a sweep was conducted to introduce variability in the imaging plane. Human acquisitions were performed free-hand, and participant data was randomly assigned to one of three sets:
- The training set (n = 14) was used to train the network using 4 anatomies (common carotid artery, thyroid gland, bicep muscle fibers, forearm). A random sub-sample of 38400 scan lines was generated (1.25% of the total available training data) and loaded for training, to comply with memory restrictions.
- The validation set (n = 5) was used to evaluate the network with the same anatomies as the training set.
- The test set (n = 3) was used to evaluate the network with three previously unseen and untrained anatomies (femoral artery, calf muscle, Achilles tendon). As phantom data for evaluation purposes, a set of 3 additional frames are acquired on a wire phantom and a LEGO model, both immersed in water, as well as a CIRS phantom (model 040GSE).

To investigate the influence of the ultrasound scanner configuration on the deepformer performances, three additional images were acquired on the CIRS phantom using different parameter settings: config 1, focus depth of 30 mm instead of 20 mm; config 2, central frequency of 5 MHz instead of 7 MHz; config 3, dynamic range of 80 instead of 50. A mechanical probe holder was used to fix the transducer in a position.

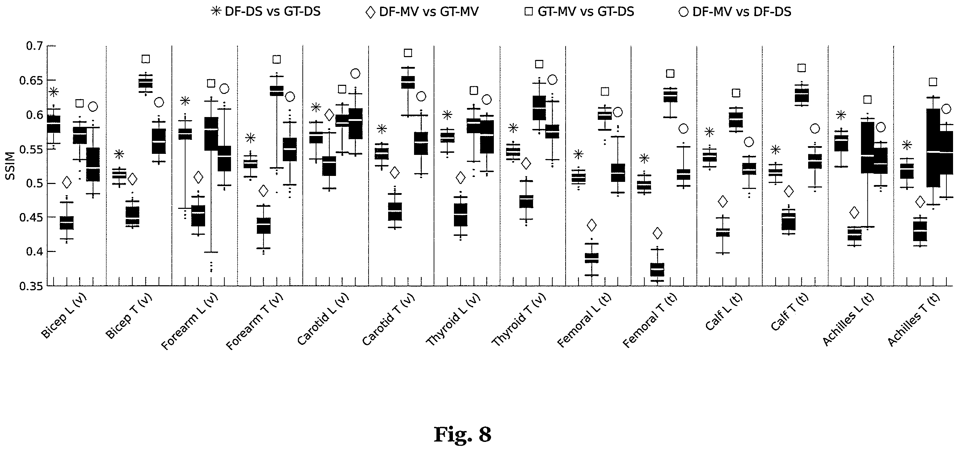

Evaluation was performed first with a focus on the suitability of deepforming for B-mode image generation. In this view, quantitative results of image similarity between fully beamformed and deepformed B-mode images are indicated for the Structural Similarity Index Measure (SSIM) in Figure 8, and the overall errors are indicated in the table below. More precisely, Fig. 8 shows the Structural Similarity Index Measure (SSIM) for the participants of the validation set (v) and the testing set (t), comparing the different beamforming strategies, where DS: delay-and-sum, MV: Minimum Variance, DF: Deepforming, GT: Ground Truth (explicit beamforming). L and T stand for Longitudinal and Transverse, respectively. Evaluation of the beamforming quality was carried out qualitatively by comparing the deepformed images with the corresponding ground truth representations, and quantitatively using the SSIM, as described in Wang, Z., Bovik, A.C., Sheikh, H.R., Simoncelli, E.P.: Image quality assessment: from error visibility to structural similarity. IEEE Transactions on Image Processing 13(4) (2004) 600-612*,* and the Mean Square Error (MSE) metric. The metric provides a strong discrimination of structural reconstruction quality, and thus lends itself well to the noisy nature of ultrasound imaging. This way, the difference in B-mode reconstructions for both MV and DS beamforming can be observed, yielding an average similarity of 0.607 ± 0.262 over the whole test set.

Shown in the below table is a comparison of the deepformed (DF) and the ground truth (GT) images, for Delay and Sum (DS) as well as Minimium Variance (MV), in the validation (v) and testing (t) datasets, using the Structural Similarity Index Measure (SSIM) and the Mean Square Error (MSE).

| | SSIM (v) | MSE (v) | SSIM (t) | MSE (t) |
|---|---|---|---|---|
| DF-DS vs GT-DS | 0.564 ± 0.222 | 25.4 ± 28.1 | 0.547 ± 0.231 | 27.2 ± 36.7 |
| DF-MV vs GT-MV | 0.497 ± 0.248 | 14.2 ± 18.2 | 0.488 ± 0251 | 14.1 ± 17.8 |
| GT-MV vs GT-DS | 0.607. ± 0.262 | 27.0 ± 33.6 | 0.589 ± 0.271 | 29.2 ± 42.4 |
| DF-MV vs DF-DS | 0.607 ± 0.224 | 17.5 ± 25.1 | 0.584 ± 0.234 | 17.6 ± 24.9 |

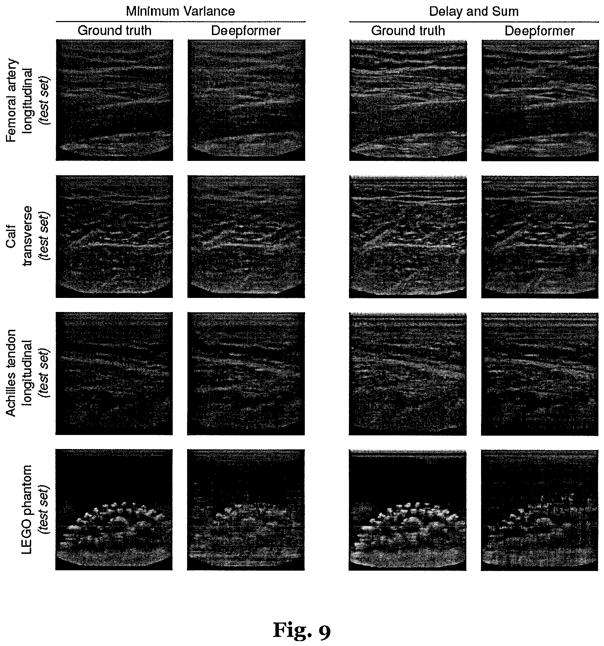

It can also be observed that while the overall similarity is lower for DF-MV vs GT-MV (MV) and DF-DS vs GT-DS (DS), they still show widely similar behavior across the test set, with overall similarities of 0.497 ± 0.248 and 0.564 ± 0.222 for MV and DS, respectively. This is also confirmed when comparing the results of the two beamforming methods qualitatively to their respective deepforming counterparts, as indicated in Figure 9. Fig. 9 shows representative examples of the similarity between deepformed images and the corresponding DS and MV ground truth. Each frame is 37.5 x 40.0 mm²

It is important to note here that the validation set represents unseen data only, and that it also includes anatomies which were not even contained in the training set, thus indicating that deepforming effectively achieves a generalization of the beamforming transformation from raw to pre-scan-converted data.

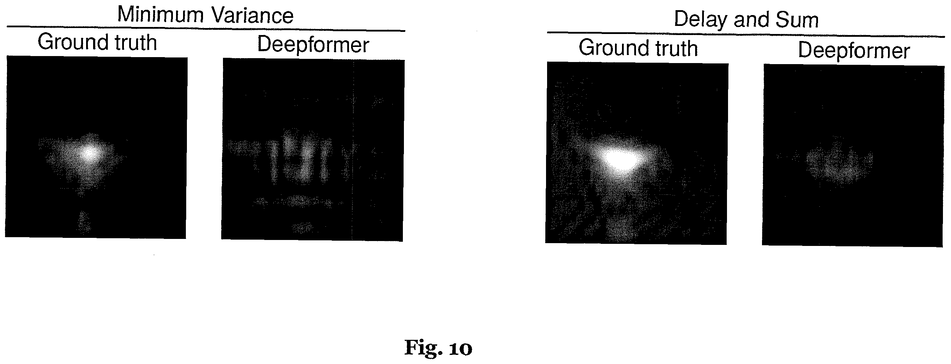

Another interesting aspect of the proposed model can be seen for the results of the LEGO phantom (Fig. 9, last row), depicting B-mode images for a structure with vastly different imaging properties (high acoustic impedance and speed of sound) to the training and validation sets. These results are confirmed by the point spread function (PSF) for the four compared methods, created by scanning a previously unseen wire phantom, as shown in Figure 10. The relatively similar PSF size between the deepformer and the ground truth suggests that image resolution is within the same range. Each image is 4 x 4 mm².

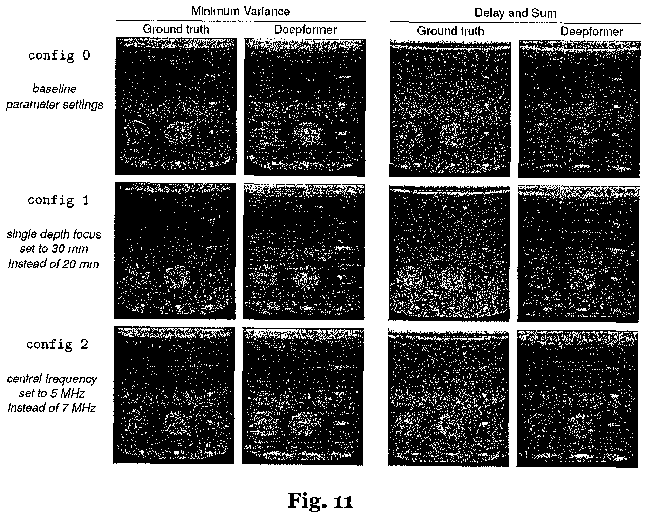

While deepforming clearly expresses artifacts unrelated to the side-lobes, the PSFs show the ability to provide a generalized mapping of beamforming methods. The evaluation of different imaging settings, config 0, config 1, and config 2 as described above, for acquisitions on a general purpose ultrasound phantom is illustrated in Figure 11. Images using the config 3 settings are not depicted because no significant change could be observed. Each frame in Fig. 11 is 37.5 x 40.0 mm². Similar to the results above, it can be seen that the trained deepformer can partially cope with deteriorated and unseen raw data parametrizations, where a change in focus and transmit frequency severely impacts received channel data.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

### Reference Signs

- 10: ultrasound transducer
- 12: transducer element
- 14: ultrasound beam
- 16: beam axis
- 18: focal region of ultrasound beam 14
- 20: electrical pulse
- 22: carrier signal
- 24: shape signal
- 26: main pulse

## Claims

1. A method of ultrasound imaging of an object using an ultrasound transducer which comprises an array of transducer elements capable of converting sound signals into electrical signals and vice versa, comprising the following steps:
A) transmitting an ultrasound beam from said ultrasound transducer into the object, by activating a first subset of said transducer elements,
B) detecting reflected signals in a time resolved manner by means of a second subset of said transducer elements, wherein timing information of a detected signal is associated with information regarding the depth where the detected signal was reflected within the object subjected to imaging, and wherein the reflected signals associated with said second subset of transducer elements resemble a set of two-dimensional ultrasound data, of which one dimension resembles the various transducer elements of said second subset and the other dimension resembles depth information,
C) converting said two-dimensional ultrasound data into a scan object using a receive beamforming procedure which accounts for differences in distance of individual transducer elements from a given site of sound reflection within the object,
repeating steps A) to C) for different choices regarding at least one of said first and second subsets and the timing of the activation of transducer elements within said first subset, thereby obtaining a plurality of scan objects, and
a step of constructing a visual image from said plurality of scan objects,
wherein said receive beamforming procedure employs a machine learning based receive beamforming model for mapping said two-dimensional ultrasound data to said scan object.

2. The method of claim 1, wherein said machine learning based receive beamforming model employs one of a deep convolutional neural network or a recurrent neural network.

3. The method of one of claims 1 and 2, wherein said receive beamforming model is an end-to-end beamforming model receiving said two-dimensional ultrasound data as an input and directly converting it into said scan object, or
wherein said receive beamforming model receives said two-dimensional ultrasound data and maps it onto a set of delay values and weight values for use in a delay-and-sum receive beamforming algorithm.

4. The method of one of the preceding claims, wherein said receive beamforming model is further configured to determine a spatial distribution of speed of sound within the object, wherein the method preferably further comprises indicating speed of sound related information in the visual image, and/or
wherein one or both of said first and second subsets of transducer elements corresponds to a number of transducer elements within a predefined aperture region centered at a given transducer element.

5. The method of one of the preceding claims, wherein said first and second subsets of transducer elements overlap with each other, wherein preferably at least 50%, more preferably at least 75% of the transducer elements in one of said first and second subsets is also part of the other one of said first and second subsets, wherein said first and second subsets are most preferably identical, or
wherein the first subset of transducer elements is larger than the second subset of transducer elements,
wherein preferably, the same first subset is combined with different second subsets, wherein preferably the first subset corresponds to the entire array of transducer elements, while different second subsets are used as receive channels for receive beamforming.

6. The method of one of the preceding claims, wherein said scan object is a scan line, representing sound reflection at various depths along a line extending from said transducer, in particular from a given transducer element of said transducer, into the object subjected to imaging, and/or
wherein said step of constructing a visual image from said plurality of scan objects comprises one or more of a demodulation, in particular a demodulation via envelope detection, a logarithmic compression and a scan conversion/re-interpolation.

7. The method of one of the preceding claims, wherein said machine learning based receive beamforming model has been trained using training data obtained with different conventional receive beamforming methods, and in particular with one or more of delay-and-sum beamforming, delay-multiply-and-sum and minimum variance beamforming,
wherein said machine learning based receive beamforming model has preferably been trained in a procedure, in which
- two or more receive beamforming procedures are carried out on the same two-dimensional ultrasound data but using different conventional receive beamforming methods, leading to a corresponding number of different scan objects, and wherein a resultant scan object is selected or derived from said plurality of different scan objects, and
- the training is carried out based on said resultant scan object.

8. The method of one of the preceding claims, wherein in step A), the activation of said first subset of transducer elements, and in particular a relative weight and/or a relative delay of their activation, is controlled
- using a transmit beamforming procedure employing a machine learning based transmit beamforming model that has been trained in combination with said machine learning based receive beamforming model, and that receives, as at least part of its input, said two-dimensional ultrasound data or said scan objects, or
- using information regarding a spatial distribution of speed of sound within the object determined by means of said receive beamforming model.

9. An apparatus for ultrasound imaging of an object, said apparatus comprising an ultrasound transducer which comprises an array of transducer elements capable of converting sound signals into electrical signals and vice versa, and a control unit, wherein said control unit is configured for controlling the apparatus to carry out the following steps:
A) transmitting an ultrasound beam from said ultrasound transducer into the object, by activating a first subset of said transducer elements,
B) detecting reflected signals in a time resolved manner by means of a second subset of said transducer elements, wherein timing information of a detected signal is associated with information regarding the depth where the detected signal was reflected within the object subjected to imaging, and wherein the reflected signals associated with said second subset of transducer elements resemble a set of two-dimensional ultrasound data, of which one dimension resembles the various transducer elements of said second subset and the other dimension resembles depth information,
C) converting said two-dimensional ultrasound data into a scan object using a receive beamforming procedure which accounts for differences in distance of individual transducer elements from a given site of sound reflection within the object,
repeating steps A) to C) for different choices regarding at least one of said first and second subsets and the timing of the activation of transducer elements within said first subset, thereby obtaining a plurality of scan objects, and
a step of constructing a visual image from said plurality of scan objects,
wherein said receive beamforming procedure employs a machine learning based receive beamforming model for mapping said two-dimensional ultrasound data to said scan object.

10. The apparatus of claim 9, wherein said machine learning based receive beamforming model employs one of a deep convolutional neural network or a recurrent neural network, and/or
wherein said receive beamforming model is an end-to-end beamforming model receiving said two-dimensional ultrasound data as an input and directly converting it into said scan object, or
wherein said receive beamforming model is configured to receive said two-dimensional ultrasound data and to map it onto a set of delay values and weight values for use in a delay-and-sum receive beamforming algorithm.

11. The apparatus of one of claims 9 or 10, wherein said receive beamforming model is further configured to determine a spatial distribution of speed of sound within the object, wherein the control unit is preferably further configured for indicating speed of sound related information in the visual image, and/or
wherein one or both of said first and second subsets of transducer elements corresponds to a number of transducer elements within a predefined aperture region centered at a given transducer element.

12. The apparatus of one of claims 9 to 11, wherein said first and second subsets of transducer elements overlap with each other, wherein preferably at least 50%, more preferably at least 75% of the transducer elements in one of said first and second subsets is also part of the other one of said first and second subsets, wherein said first and second subsets are most preferably identical, or
wherein the first subset of transducer elements is larger than the second subset of transducer elements,
wherein preferably, the same first subset is combined with different second subsets, wherein preferably the first subset corresponds to the entire array of transducer elements, while different second subsets are used as receive channels for receive beamforming.

13. The apparatus of one of claims 9 to 12, wherein said scan object is a scan line, representing sound reflection at various depths along a line extending from said transducer, in particular from a given transducer element of said transducer, into the object subjected to imaging, and/or
wherein said step of constructing a visual image from said plurality of scan objects comprises one or more of a demodulation, in particular a demodulation via envelope detection, a logarithmic compression and a scan conversion/re-interpolation.

14. The apparatus of one of claims 9 to 13, wherein said machine learning based receive beamforming model has been trained, or is obtainable by training using training data obtained with different conventional receive beamforming methods, and in particular with one or more of delay-and-sum beamforming, delay-multiply-and-sum and minimum variance beamforming,
wherein said machine learning based receive beamforming model has preferably been trained in a procedure or is obtainable by training in a procedure, in which
- two or more receive beamforming procedures are carried out on the same two-dimensional ultrasound data but using different conventional receive beamforming methods, leading to a corresponding number of different scan objects, and wherein a resultant scan object is selected or derived from said plurality of different scan objects, and
- the training is carried out based on said resultant scan object.

15. The apparatus of one of claims 9 to 14, wherein said control unit is configured to control in step A) the activation of said first subset of transducer elements, and in particular a relative weight and/or a relative delay of their activation,
- using a transmit beamforming procedure employing a machine learning based transmit beamforming model that has been trained in combination with said machine learning based receive beamforming model, and that receives, as at least part of its input, said two-dimensional ultrasound data or said scan objects, or
- using information regarding a spatial distribution of speed of sound within the object determined by means of said receive beamforming model.
